# EUROPEAN PATENT SPECIFICATION

(11) **EP 0 689 438 B1**
(45) Date of publication and mention of the grant of the patent: **04.06.2003**
(21) Application number: 94911944.0
(22) Date of filing: 18.03.1994
(51) Int. Cl.: A61K 31/485, A61K 9/14, A61P 25/16

(54) **PHARMACEUTICAL COMPOSITIONS FOR INTRANASAL ADMINISTRATION OF APOMORPHINE**
PHARMAZEUTISCHE ZUSAMMENSETZUNGEN ZUR INTRANASALEN VERABREICHUNG VON APOMORPHIN
COMPOSITIONS PHARMACEUTIQUES POUR L'ADMINISTRATION INTRANASALE D'APOMORPHINE

(30) Priority: 26.03.1993 BE 9300297; 26.03.1993 BE 9300298; 26.03.1993 BE 9300299
(43) Date of publication of application: 03.01.1996
(60) Divisional application: 98109002.0
(73) Proprietor: Merkus, Franciscus Wilhelmus Henricus Maria, B-2460 Kasterlee (BE)
(72) Inventor: Merkus, Franciscus Wilhelmus Henricus Maria, B-2460 Kasterlee (BE)
(74) Representative: Jump, Timothy John Simon
(86) International application number: PCT/EP94/00891
(87) International publication number: WO 94/022445

(56) References cited:
- EP-A- 0 094 157
- EP-A- 0 205 282
- EP-A- 0 463 653
- EP-A- 0 475 482
- WO-A-91/09599
- WO-A-93/15737
- DE-A- 4 207 922
- GB-A- 1 592 563
- US-A- 5 169 849
- DEUTSCHE APOTHEKER ZEITUNG vol. 130, no. 44 , 1 November 1990 pages 2411 - 15 HERMENS ET AL 'Der Einfluss von Arzneimitteln auf die Bewegung nasaler Flimmerhaare'

## Description

This invention is related to pharmaceutical compositions for nasal administration of apomorphine and to methods of administering such compositions.

Apomorphine is a very potent dopamine agonist. It is used as an adjunctive medication in the treatment of Parkinson's disease, complicated by motor fluctuations. Recently, encouraging results have been reported on the intranasal application of apomorphine in patients with Parkinson's disease to relieve "off-period" symptoms in patients with response fluctuations (T. van Laar et al, Arch. Neurol. 1992; 49: 482-484). The intranasal applied apomorphine, used by these authors, consisted of an aqueous solution of apomorphine HCl 10 mg/ml. This formulation is also used for parenteral application and is published in different Pharmacopoeia's.

The exact nasal formulation used in the study by T. van Laar et al (1992) was:

| | |
|---|---|
| Apomorphine HCl 0.5 H₂O | 1g |
| Sodium metabisulphite | 0.100 g |
| Sodium EDTA | 0.010 g |
| NaCl | 0.600 g |
| Benzalkonium Chloride | 0.01 % |
| NaH₂PO₄ . 2H₂O | 0.150 g |
| Na₂HPO₄ . 2H₂O | 0.050 g |
| NaOH 1 M to adjust pH at 5.8 purified water to 100 ml (from Pharm. Weekblad 1991; 126: 1113-1114) | |

By a metered dose nebulizer a dose of 1 mg apomorphine HCl (0.1 ml of the solution) was delivered with each nasal application by puff to the patients. A great disadvantage of this aqueous solution is the instability of the apomorphine.

EP 0475482-A discloses dry formulations each comprising a water soluble acid addition salt of a poorly soluble basic compound in admixture with microcrystalline cellulose, lactose, or calcium hydrogen phosphate, and a water soluble alkaline stabilizer. Apomorphine is among the exemplified basic compounds. The proposed formulations are said to be rendered more stable and "rugged" and, hence, better suited to preparation by wet or dry granulation, by the alkaline stabilizer.

EP 0094157-A discloses a composition comprising a hydrophillic drug, such as a physiologically active polypeptide, polysaccharide, nucleic acid, aminoglycoside antibiotic, or beta-lactam antibiotic, with a cyclodextrin, preferably an alpha-cyclodextrin, for nasal, vaginal or rectal administration. Apomorphine is not mentioned in this document.

EP 0463653-A discloses the use of cyclodextrins to reduce inflammation and other side effects caused by absorption enhancing agents, when the latter are used in formulations for drug delivery by the intranasal route. The absorption enhancing agents include chelating agents, fatty acids, bile acid salts, surfactants, fusidic acid and its derivatives, lysophosphatides and cyclic peptide antibiotics. Although a substantial list of drugs is provided in this document, apomorphine is not mentioned.

An object of the invention is to provide a nasal formulation of apomorphine with an improved bioavailability and stability of apomorphine.

According to a first aspect of the invention, there is provided a powdered pharmaceutical composition for nasal administration comprising apomorphine, or an apomorphine salt, and an excipient, wherein the excipient comprises a saccharide, or a sugar alcohol.

Preferred compositions in accordance with the first aspect of the invention are defined in claims 2-10 appended hereto.

In a second aspect, the present invention provides the use of apomorphine, or a salt thereof, in the preparation of a medicament for treating Parkinson's disease by administering the medicament to the nasal mucosa, wherein the medicament comprises a powdered pharmaceutical composition in accordance with the first aspect of the invention.

The term "cyclodextrin" refers to cyclic oligosaccharides, like α-, β- and γ-cyclodextrin and their derivatives, preferably β-cyclodextrin and its derivatives, preferably methylated β-cyclodextrin, with a degree of CH₃-substitution between 0.5 and 3.0, more preferably between 1.7 and 2.1. The term "saccharide" refers to disaccharides, like lactose, maltose, saccharose and also refers to polysaccharides, like dextrans, with an average molecular weight between 10.000 and 100.000, preferably 40.000 and 70.000. The term "sugar alcohols" refers to mannitol and sorbitol.

Powder formulations in accordance with the invention show a surprisingly high bioavailability and superior stability of the apomorphine.

In addition, powder formulations have the advantage that no preservatives are necessary. Preservatives are known to decrease the ciliary movement, which may be harmful in chronic nasal medication (Hermens W.A.J.J. and Merkus F.W.H.M., Pharm. Res. 1987; 4: 445-449).

Nasal powder compositions can be made by mixing the active agent and the excipient, both possessing the desired particle size. Other methods to make a suitable powder formulation can be selected. Firstly, a solution of the active agent, a saccharide and/or a sugar alcohol is made, followed by precipitation, filtration and pulverization. It is also possible to remove the solvent by freeze drying, followed by pulverization of the powder in the desired particle size by using conventional techniques, known from the pharmaceutical literature. The final step is size classification for instance by sieving, to get particles that are less than 100 micrometers in diameter, preferably between 50 and 100 micrometers in diameter. Powders can be administered using a nasal insufflator. Powders may also be administered in such a manner that they are placed in a capsule. The capsule is set in an inhalation or insufflation device. A needle is penetrated through the capsule to make pores at the top and the bottom of the capsule and air is sent to blow out the powder particles. The powder formulation can also be administered in a jet-spray of an inert gas or suspended in liquid organic fluids.

The amount of a powdered nasal formulation is generally between 1 and 15 mg and, preferably, about 5 to 10 mg per nostril. Doses of apomorphine in the pharmaceutical composition of the present invention, suitable in the treatment of Parkinson's disease, are generally in the range of 0.1 to 2 mg per nostril.

The following examples illustrate the present invention in more detail:

### EXAMPLE 1A (powder)

| | |
|---|---|
| Apomorphine base | 1 mg |
| Methyl-β-cyclodextrin D.S. 2.1 | 5 mg |
| Mannitol | 4 mg |
| 10 mg powder = 1 mg Apomorphine | |

### EXAMPLE 1B (powder)

| | |
|---|---|
| Apomorphine HCl | 2 mg |
| Mannitol | 18 mg |
| 20 mg powder = 2 mg Apomorphine HCl | |

### EXAMPLE 1C (powder)

| | |
|---|---|
| Apomorphine HCl | 1 mg |
| Dextran (average M.W. 70.000) | 9 mg |
| 10 mg powder - 1 mg Apomorphine HCl | |

## Claims

1. A powdered pharmaceutical composition for nasal administration comprising apomorphine, or an apomorphine salt, and an excipient, wherein the excipient comprises a saccharide and/or a sugar alcohol.

2. A powdered pharmaceutical composition as claimed in claim 1, wherein the apomorphine salt is apomorphine hydrochloride.

3. A powdered pharmaceutical composition as claimed in claim 1 or claim 2, wherein the saccharide is a cyclodextrin.

4. A powdered pharmaceutical composition as claimed in claim 3, wherein the cyclodextrin is α-, β- or γ-cyclodextrin or a derivative of α-, β- or γ-cyclodextrin.

5. A powdered pharmaceutical composition as claimed in claim 3 or claim 4, wherein the cyclodextrin is β-cyclodextrin or a derivative.

6. A powdered pharmaceutical composition as claimed in claim 5, wherein the derivative of β-cyclodextrin is a methylated β-cyclodextrin with a degree of substitution between 0.5 and 3.0.

7. A powdered pharmaceutical composition as claimed in any of claims 3-6, wherein the excipient comprises another saccharide.

8. A powdered pharmaceutical composition as claimed in claims 1, 2 or 7, wherein the saccharide is a disaccharide or a polysaccharide.

9. A powdered pharmaceutical composition as claimed in claim 8, wherein the saccharide is lactose.

10. A powdered pharmaceutical composition as claimed in claim 8, wherein the saccharide is a dextran having an average molecular weight between 10,000 and 100,000.

11. A powdered pharmaceutical composition as claimed in claim 10, wherein the dextran has an average molecular weight of between 40,000 and 70,000.

12. A powdered pharmaceutical composition as claimed in any of the preceding claims wherein the sugar alcohol is mannitol or sorbitol.

13. A powdered pharmaceutical composition as claimed in any of the preceding claims, wherein the particles of the powder have a diameter between 50-100 micrometers.

14. A process for preparing a powdered nasal pharmaceutical composition as claimed in any of claims 1-13, which process comprises combining apomorphine or a salt with an excipient comprising a saccharide and/or a sugar alcohol.

15. Use of apomorphine, or a salt thereof, in the preparation of a medicament for treating Parkinson's disease by administering the medicament to the nasal mucosa, wherein the medicament comprises a powdered pharmaceutical composition as claimed in any of claims 1-13.

## Patentansprüche

1. Pulverförmige pharmazeutische Zusammensetzung zur nasalen Verabreichung umfassend Apomorphin oder ein Apomorphinsalz, und einen Träger, wobei der Träger ein Saccharid und/oder einen Zuckeralkohol umfaßt.

2. Pulverförmige pharmazeutische Zusammensetzung nach Anspruch 1, wobei das Apomorphinsalz Apomorphinhydrochlord ist.

3. Pulverförmige pharmazeutische Zusammensetzung nach Anspruch 1 oder 2, wobei das Saccharid ein Cyclodextrin ist.

4. Pulverförmige pharmazeutische Zusammensetzung nach Anspruch 3, wobei das Cyclodextrin ein α-, β- oder γ-Cyclodextrin oder ein Derivat von α-, β- oder γ-Cyclodextrin ist.

5. Pulverförmige pharmazeutische Zusammensetzung nach Anspruch 3 oder Anspruch 4, wobei das Cyclodextrin β-Cyclodextrin oder ein Derivat ist.

6. Pulverförmige pharmazeutische Zusammensetzung nach Anspruch 5, wobei das Derivat β-Cyclodextrin ein methyliertes β-Cyclodextrin mit einem Substitutionsgrad zwischen 0,5 und 3,0 ist.

7. Pulverförmige pharmazeutische Zusammensetzung nach einem der Ansprüche 3-6, wobei der Träger ein weiteres Saccharid umfaßt.

8. Pulverförmige pharmazeutische Zusammensetzung Anspruch 1, 2 oder 7, wobei das Saccharid ein Disaccharid oder ein Polysaccharid ist.

9. Pulverförmige pharmazeutische Zusammensetzung nach Anspruch 8, wobei das Saccharid Laktose ist.

10. Pulverförmige pharmazeutische Zusammensetzung nach Anspruch 8, wobei das Saccharid ein Dextran mit einem durchschnittlichen Molekulargewicht zwischen 10000 und 100000 ist.

11. Pulverförmige pharmazeutische Zusammensetzung nach Anspruch 10, wobei das Dextran ein durchschnittliches Molekulargewicht zwischen 40000 und 70000 aufweist.

12. Pulverförmige pharmazeutische Zusammensetzung nach einem der vorhergehenden Ansprüche, wobei der Zuckeralkohol Mannitol oder Sorbitol ist.

13. Pulverförmige pharmazeutische Zusammensetzung nach einem der vorhergehenden Ansprüche, wobei die Partikel des Pulvers einen Durchmesser zwischen 50-100 Mikrometern aufweisen.

14. Verfahren zur Herstellung einer pulverförmigen nasalen pharmazeutischen Zusammensetzung nach einem der Ansprüche 1-13, wobei das Verfahren das Kombinieren von Apomorphin oder einem Salz mit einem Träger, umfassend ein Saccharid und/oder einen Zuckeralkol, umfaßt.

15. Verwendung von Apomorphin, oder einem Salz davon, zur Herstellung eines Arzneimittels zur Behandlung der Parkinson-Krankheit durch Verabreichung des Arzneimittels an die nasale Mukosa, wobei das Arzneimittel eine pulverförmige pharmazeutische Zusammensetzung nach einem der Ansprüche 1-13 umfaßt.

## Revendications

1. Une composition pharmaceutique pulvérisée destinée à l'administration nasale et comprenant de l'apomorphine ou un sel d'apomorphine et un excipient, composition dans laquelle l'excipient est constitué par un saccharide et/ou un alcool de la série des sucres.

2. Une composition pharmaceutique pulvérisée selon la revendication 1, dans laquelle le sel d'apomorphine est le chlorhydrate d'apomorphine.

3. Une composition pharmaceutique pulvérisée selon la revendication 1 ou la revendication 2, dans laquelle le saccharide est une cyciodextrine.

4. Une composition pharmaceutique pulvérisée selon la revendication 3, dans laquelle la cyclodextrine est l'α-, la β-ou la γ-cyclodextrine ou un dérivé d'α-, de β-ou de γ-cyclodextrine.

5. Une composition pharmaceutique pulvérisée selon la revendication 3 ou la revendication 4, dans laquelle la cyclodextrine est la β-cyclodextrine ou un de ses dérivés.

6. Une composition pharmaceutique pulvérisée selon la revendication 5, dans laquelle le dérivé de β-cyclodextrine est une β-cyclodextrine méthylée avec un degré de substitution compris entre 0,5 et 3,0.

7. Une composition pharmaceutique pulvérisée selon l'une des revendications 3 à 6, dans laquelle l'excipient comprend un autre saccharide.

8. Une composition pharmaceutique pulvérisée selon les revendications 1, 2 ou 7, dans laquelle le saccharide est un disaccharide ou un polysaccharide.

9. Une composition pharmaceutique pulvérisée selon la revendication 8, dans laquelle le saccharide est le lactose.

10. Une composition pharmaceutique pulvérisée selon la revendication 8, dans laquelle le saccharide est un dextrane ayant un poids moléculaire moyen compris entre 10 000 et 100 000.

11. Une composition pharmaceutique pulvérisée selon la revendication 10, dans laquelle le dextrane a un poids moléculaire moyen compris entre 40 000 et 70 000.

12. Une composition pharmaceutique pulvérisée selon l'une quelconque des revendications précédentes, dans laquelle l'alcool de la série des sucres est le mannitol ou le sorbitol.

13. Une composition pharmaceutique pulvérisée selon l'une quelconque des revendications précédentes, dans laquelle les particules de la poudre ont un diamètre compris entre 50 et 100 micromètres.

14. Un procédé pour la préparation d'une composition pharmaceutique nasale pulvérisée selon l'une quelconque des revendications 1 à 13, procédé consistant à combiner de l'apomorphine ou un sel d'apomorphine avec un excipient constitué par un saccharide et/ou un alcool de la série des sucres.

15. Utilisation de l'apomorphine ou d'un sel de celle-ci dans la préparation d'un médicament destiné au traitement de la maladie de Parkinson par administration du médicament sur la muqueuse nasale, utilisation dans laquelle le médicament consiste en une composition pharmaceutique pulvérisée selon l'une quelconque des revendications 1 à 13.
